# EUROPEAN PATENT APPLICATION

(11) **EP 4 230 234 A1**
(43) Date of publication of application: **23.08.2023**
(21) Application number: 21905118.2
(22) Date of filing: 27.08.2021
(51) Int. Cl.: A61M 1/00, A61M 3/04

(54) **UTERINE CAVITY PERFUSION SYSTEM AND PERFUSION METHOD**

(30) Priority: 17.12.2020 CN 202011494472
(71) Applicant: Shanghai Yodo Medical Technologies AG Co., Ltd, Shanghai 201318 (CN)
(72) Inventor: YANG, Xiang, Shanghai 201318 (CN); WANG, Chenfeng, Shanghai 201318 (CN); HAO, Jinzheng, Shanghai 201318 (CN); WU, Hai, Shanghai 201318 (CN)
(74) Representative: Ridderbusch, Oliver
(86) International application number: PCT/CN2021/115016
(87) International publication number: WO 2022/127185

(57) **Abstract**

The present invention discloses a uterine cavity perfusion system and a uterine cavity perfusion method. The uterine cavity perfusion system includes a uterine cavity distention assembly, a suction assembly and a control assembly, wherein the uterine cavity distention assembly includes a uterine cavity distention tube, a saline storage container and a uterine cavity distention pump; the uterine cavity distention tube is connected to the saline storage container and a uterine cavity; the uterine cavity distention pump is installed on the uterine cavity distention tube, and is used for driving liquid in the saline storage container to enter the uterine cavity; the suction assembly includes a suction tube, a waste liquid collection container and a driving member; the suction tube is connected to the waste liquid collection container, and the driving member is installed on the suction tube and used for driving liquid in the uterine cavity to enter the waste liquid collection container; the control assembly includes a controller; the controller is connected to the uterine cavity distention pump and the driving member respectively; and the controller is used for coordinating the work of the uterine cavity distention pump and the driving member so that a pressure of the liquid in the uterine cavity can be stable. According to the uterine cavity perfusion system and the uterine cavity perfusion method, a pressure value in the uterine cavity can be maintained within a preset range.

## Description

### Technical Field

The present invention relates to the medical field for uterine cavities, and further relates to a uterine cavity perfusion system and a uterine cavity perfusion method.

### Background Art

A uterine cavity perfusion system is a circulating perfusion system that drives a liquid into and out of a uterine cavity during a uterine cavity surgery and is thus an essential system for the uterine cavity surgery.

At present, the existing uterine cavity perfusion systems all use a natural outflow mode for circulating perfusion, but some use a mode of driving a liquid to flow by a pump for circulating perfusion. It should be pointed out that a volume of a uterine cavity of an adult woman is generally about 5 ml, and a pump-driven mode is used for circulating perfusion, but with a change in a working state of a negative pressure pump, it is easy to lead to a sudden increase or decrease in a liquid pressure in the uterine cavity, and then lead to a large pressure in the uterine cavity or the collapse of the uterine cavity, thereby affecting the surgical field of view and surgical safety.

In summary, improvements to the traditional uterine cavity perfusion systems are needed.

### Summary of the Invention

In response to the above technical problems, an object of the present invention is to provide a uterine cavity perfusion system and a uterine cavity perfusion method. The uterine cavity perfusion system adopts a uterine cavity distention system and a suction system to work in cooperation with each other, so as to ensure that the uterine cavity distention system can make a cooperative response in time while the suction system works, so that a pressure value in the uterine cavity is maintained in a preset range.

In order to achieve the above object, an object of the present invention is to provide a uterine cavity perfusion system, comprising:
a uterine cavity distention assembly, comprising a uterine cavity distention tube, a saline storage container and a uterine cavity distention pump, wherein the uterine cavity distention tube is connected to the saline storage container and a uterine cavity, and the uterine cavity distention pump is installed on the uterine cavity distention tube and used for driving liquid in the saline storage container to enter the uterine cavity;
a suction assembly, comprising a suction tube, a waste liquid collection container and a driving member, wherein the suction tube is connected to the waste liquid collection container, and the driving member is installed on the suction tube and used for driving liquid in the uterine cavity to enter the waste liquid collection container; and
a control assembly, comprising a controller, wherein the controller is connected to the uterine cavity distention pump and the driving member respectively, and the controller is used for coordinating the work of the uterine cavity distention pump and the driving member so that a pressure of the liquid in the uterine cavity is stable.

Preferably, the controller, prior to controlling the driving member to drive the liquid in the uterine cavity to leave through the suction tube, controls the uterine cavity distention pump to pre-deliver a predetermined amount of liquid into the uterine cavity, so as to prevent the collapse of the uterine cavity caused by fast outflow of the liquid in the uterine cavity.

Preferably, the control assembly further comprises a uterine cavity distention pump pressure sensor and a driving member pressure sensor, wherein the uterine cavity distention pump pressure sensor is installed on the uterine cavity distention tube and used for detecting a liquid pressure in the uterine cavity distention tube; and the driving member pressure sensor is installed on the suction tube and used for detecting a liquid pressure in the suction tube.

Preferably, the driving member comprises a suction pump; the waste liquid collection container comprises a first waste liquid collection container; and the suction pump is disposed between the first waste liquid collection container and the uterine cavity and used for driving the liquid in the suction tube to flow from the uterine cavity to the first waste liquid collection container.

Preferably, the driving member comprises a vacuum suction pump; the waste liquid collection container comprises a second waste liquid collection container; the second waste liquid collection container is disposed between the vacuum suction pump and the uterine cavity; and the vacuum suction pump is used for sucking a gas in the second waste liquid collection container to generate a negative pressure in the second waste liquid collection container, thereby driving the liquid in the uterine cavity to enter.

Preferably, the driving member comprises a vacuum suction pump; the waste liquid collection container comprises a second waste liquid collection container; the vacuum suction pump is communicated with the second waste liquid collection container and used for sucking a liquid in the second waste liquid collection container; the suction assembly further comprises a three-way valve; the suction pump is communicated with the three-way valve; and the three-way valve is also connected to the second waste liquid collection container.

Preferably, the driving member further comprises a pinch valve; and the pinch valve is connected between the second waste liquid collection container and the uterine cavity and used for controlling the connection and disconnection of the suction tube between the second waste liquid collection container and the uterine cavity.

Preferably, the driving member further comprises a pinch valve; the suction assembly further comprises a three-way valve; one end of the pinch valve is connected to the first waste liquid collection container, and the other end of the pinch valve is connected to the three-way valve; and the three-way valve is also connected to the suction pump.

Preferably, the driving member further comprises a pinch valve; the waste liquid collection container further comprises a third waste liquid collection container; the suction assembly further comprises a three-way valve; one end of the pinch valve is connected to the third waste liquid collection container, and the other end of the pinch valve is connected to the three-way valve; and the three-way valve is also connected to the second waste liquid collection container.

According to another aspect of the present invention, the present invention further provides a uterine cavity perfusion method, comprising:
injecting a preset amount of liquid into a uterine cavity in advance through a uterine cavity distention assembly while a liquid in the uterine cavity needs to be discharged, and then discharging the liquid in the uterine cavity through a suction assembly, wherein the uterine cavity distention assembly and the suction assembly work in cooperation with each other.

Preferably, a corresponding control instruction is sent to a controller while the waste liquid in the uterine cavity needs to be discharged; the controller receives the corresponding control instruction, and then controls a uterine cavity distention pump of the uterine cavity distention assembly to deliver a first preset amount of liquid into the uterine cavity, wherein the first preset amount corresponds to a suction pump of the suction assembly; and the controller then controls the suction pump to work, driving the liquid in the uterine cavity to enter the waste liquid collection container through the suction tube.

Preferably, the uterine cavity perfusion method, the use of the uterine cavity perfusion system comprises the following steps:
pressing a liquid discharge button to send a corresponding control instruction to the controller;
controlling, by the controller, the uterine cavity distention pump of the uterine cavity distention assembly to deliver the first preset amount of liquid into the uterine cavity in response to receiving the corresponding control instruction; and
controlling, by the controller, the suction pump of the suction assembly to work after a first preset time, so as to drive the liquid in the uterine cavity to enter the first waste liquid collection container through the suction tube.

Preferably, a corresponding control instruction is sent to the controller when the liquid in the uterine cavity needs to be discharged; the controller receives the corresponding control construction, and then controls the uterine cavity distention pump of the uterine cavity distention assembly to pre-start to inject a second preset amount of liquid into the uterine cavity, wherein the second preset amount corresponds to a response speed of a vacuum suction pump; and the controller then controls the vacuum suction pump to work to suck a gas in a second waste liquid collection container, thereby forming a negative pressure in the second waste liquid collection container so as to drive the liquid in the uterine cavity to enter under the action of the gas pressure.

Preferably, the uterine cavity perfusion method, the use of the uterine cavity perfusion system comprises the following steps:
pressing a liquid discharge button to send a corresponding control instruction to the controller;
controlling, by the controller, the uterine cavity distention pump of the uterine cavity distention assembly to deliver the second preset amount of liquid into the uterine cavity in response to receiving the corresponding control instruction; and
controlling, by the controller, the vacuum suction pump of the suction assembly to work after a second preset time to suck the gas in the second waste liquid collection container, thereby forming a negative pressure in the second waste liquid collection container so as to drive the liquid in the uterine cavity to enter under the action of the gas pressure.

The uterine cavity perfusion system and the uterine cavity perfusion method provided by the present invention have at least one of the following beneficial effects:
1. according to the uterine cavity perfusion system and the uterine cavity perfusion method provided by the present invention, the uterine cavity perfusion system and the uterine cavity perfusion method can control the uterine cavity distention assembly to inject a preset amount of liquid into the uterine cavity in advance prior to discharging the liquid in the uterine cavity, thereby preventing the collapse of the uterine cavity caused by the sudden discharge of the liquid in the uterine cavity;
2. according to the uterine cavity perfusion system and the uterine cavity perfusion method provided by the present invention, the uterine cavity perfusion system uses the vacuum suction pump to generate a negative pressure, and the liquid in the uterine cavity is discharged by negative pressure suction, which can effectively eliminate the obstruction of the suction tube between the uterine cavity and the waste liquid collection container, and effectively maintain the working stability of the uterine cavity perfusion system; and
3. according to the uterine cavity perfusion system and the uterine cavity perfusion method provided by the present invention, the uterine cavity perfusion system can quickly switch the modes of liquid discharge in the uterine cavity through a three-way valve, and can provide a variety of liquid discharge modes, thereby achieving a simple structure and easy control.

### Brief Description of the Drawings

The preferred embodiments are described clearly and understandably in conjunction with accompanying drawings, in order to further explain the above characteristics, technical features, advantages and their implementations of the present invention.
FIG. 1 is a schematic structure diagram of a uterine cavity perfusion system according to a first preferred embodiment of the present invention;
FIG. 2 is a schematic structure diagram of a uterine cavity perfusion system according to a second preferred embodiment of the present invention;
FIG. 3 is a schematic structure diagram of a uterine cavity perfusion system according to a third preferred embodiment of the present invention;
FIG. 4 is a schematic structure diagram of a uterine cavity perfusion system according to a fourth preferred embodiment of the present invention;
FIG. 5 is a schematic structure diagram of a uterine cavity perfusion system according to a fifth preferred embodiment of the present invention; and
FIG. 6 is a schematic structure diagram of a uterine cavity perfusion system according to a sixth preferred embodiment of the present invention.

Symbols represents the following components:
1-uterine cavity distention assembly; 11-uterine cavity distention tube; 12-saline storage container; 13-uterine cavity distention pump; 2-suction assembly; 21-suction tube; 22-waste liquid collection container; 221-first waste liquid collection container; 222-second waste liquid collection container; 23-driving member; 231-suction pump; 232-vacuum suction pump; 233-pinch valve; 24-three-way valve; 25-filtering member; 3-control assembly; 32-uterine cavity distention pump pressure sensor; 33-driving member pressure sensor; and 4-uterine cavity.

### Detailed Descriptions of the Preferred Embodiments

In order to more clearly illustrate embodiments of the present invention or technical solutions in the prior art, the detailed implementations of the present invention will be described below by reference to the accompanying drawings. Apparently, the accompanying drawings in the following description show merely some embodiments of the present invention, and a person of ordinary skill in the art may still derive other drawings from these accompanying drawings without creative efforts, and derive other implementations.

For the sake of brevity, each drawing only schematically represents the parts related to the present invention, but they do not represent their actual structures as products. Further, in order to make the drawings concise and easy to understand, for parts in some drawings that have the same structure or function, only one of the parts is schematically depicted, or only marked. "One" herein means a case of not only "only this one", but also "more than one".

It should also be further understood that the term "and/or" as used in the description of the present application and the accompanying claims refers to any combination of one or more of the associated listed items and all possible combinations, and includes these combinations.

In the description of the present invention, it should be noted that, unless otherwise definitely specified and limited, the terms "mounted", "connected with each other", and "connected to/with" need to be broadly understood, for example, connection may be fixed connection, or detachable connection or integrated connection; or may be mechanical connection or electric connection; or may be direct connection, or indirect connection via an intermediation, or communication of inner parts of two elements. A person of ordinary skill in the art can understand the specific meaning of the above terms in the present invention in accordance with specific conditions.

Furthermore, in the description of the present application, the terms "first" and "second" are used for a distinguishing description only and are not to be construed as indicating or implying relative importance.

### Embodiment 1:

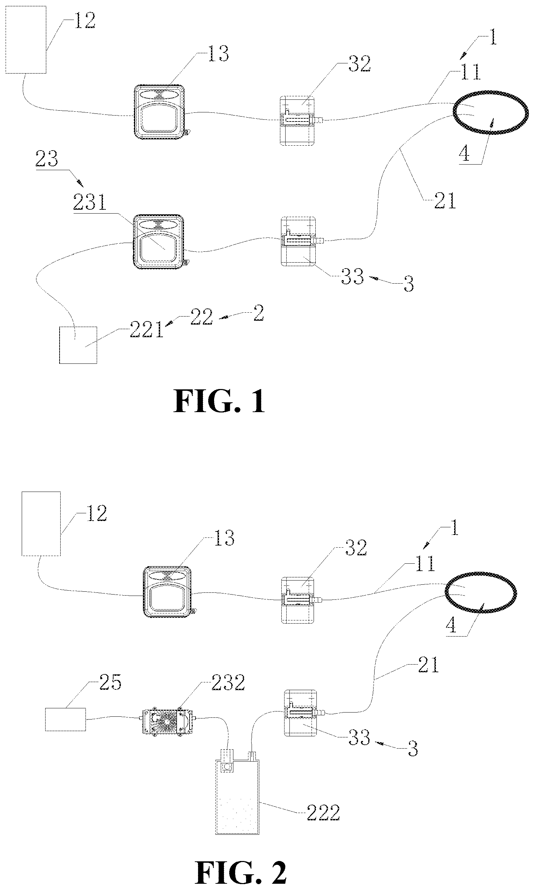

Referring to FIG. 1 of the description, the present invention provides a uterine cavity perfusion system. The uterine cavity perfusion system is described to be capable of coordinating the work of a uterine cavity distention assembly and a suction assembly during a working process of the uterine cavity distention assembly and the suction assembly, so as to ensure that a pressure value in a uterine cavity is maintained within a preset range, thereby preventing a sudden increase in a pressure in the uterine cavity caused by a sudden increase in the liquid in the uterine cavity or the collapse of the uterine cavity caused by a sudden decrease in the liquid in the uterine cavity.

Referring to FIG. 1 of the description, specifically, the uterine cavity perfusion system includes a uterine cavity distention assembly 1, a suction assembly 2, and a control assembly 3. The uterine cavity distention assembly 1 includes a uterine cavity distention tube 11, a saline storage container 12 and a uterine cavity distention pump 13; the uterine cavity distention tube 11 is connected to the saline storage container 12 and a uterine cavity 4; the uterine cavity distention pump 13 is installed on the uterine cavity distention tube 11 and used for driving a liquid in the saline storage container 12 to enter the uterine cavity 4; the suction assembly 2 includes a suction tube 21, a waste liquid collection container 22 and a driving member 23; the suction tube 21 is connected to the waste liquid collection container 22, and the driving member 23 is installed on the suction tube 21 and used for driving the liquid in the uterine cavity 4 to enter the waste liquid collection container 22; the control assembly 3 includes a controller; the controller is connected to the uterine cavity distention pump 13 and the driving member 23 respectively; and the controller is used for coordinating the work of the uterine cavity distention pump 13 and the driving member 23 so that a pressure of the liquid in the uterine cavity 4 can be stable.

It should be pointed out that the control assembly 3 of the uterine cavity perfusion system provided by the present invention can control the uterine cavity distention assembly 1 and the suction assembly 2 to work in cooperation with each other, so that the liquid pressure in the uterine cavity 4 is maintained within a preset pressure value range, e.g., not less than 80% of a set value, but not higher than 120% of the set value. Therefore, the case of the collapse resulting from a sudden decrease in pressure in the uterine cavity 4 caused by a sudden outflow of the liquid in the uterine cavity 4, or a sudden increase in pressure caused by a sudden increase in the liquid in the uterine cavity 4 can be effectively prevented.

Further, in the uterine cavity perfusion system provided by the present invention, the controller, prior to controlling the driving member 23 to drive the liquid in the uterine cavity 4 to leave through the suction tube 21, controls the uterine cavity distention pump 11 to pre-deliver a predetermined amount of liquid into the uterine cavity 4 so as to prevent the collapse of the uterine cavity 4 caused by the fast outflow of the liquid in the uterine cavity 4. In other words, in the uterine cavity perfusion system provided by the present invention, an early warning is provided to the uterine cavity distention pump 13 before the liquid in the uterine cavity 4 is discharged through the driving member 23, and a preset amount of liquid is injected into the uterine cavity 4 in advance through the uterine cavity distention pump 13, thereby preventing a sudden decrease in pressure in the uterine cavity 4.

The control assembly 3 further includes a uterine cavity distention pump pressure sensor 32 and a driving member pressure sensor 33, wherein the uterine cavity distention pump pressure sensor 32 is installed on the uterine cavity distention tube 11 and used for detecting a liquid pressure in the uterine cavity distention tube 11; and the driving member pressure sensor 33 is installed on the suction tube and used for detecting a liquid pressure in the suction tube 21.

The uterine cavity distention pump pressure sensor 32 and the driving member pressure sensor 33 are connected to the controller, respectively. The uterine cavity distention pump pressure sensor 32 and the driving member pressure sensor 33 can transmit detected pressure data to the controller, respectively. The controller can control the uterine cavity distention pump 13 and the driving member 23 to work in cooperation with each other based on the detection data of the uterine cavity distention pump pressure sensor 32 and the driving member pressure sensor 33.

Referring to FIG. 1 of the description, the driving member 23 includes a suction pump 231; the waste liquid collection container 22 includes a first waste liquid collection container 221; and the suction pump 231 is disposed between the first waste liquid collection container 221 and the uterine cavity 4 and used for driving the liquid in the suction tube 21 to flow from the uterine cavity 4 to the first waste liquid collection container 221.

In the working process of the uterine cavity perfusion system in the present preferred embodiment, a corresponding control instruction is sent to the controller while the waste liquid in the uterine cavity 4 needs to be discharged; the controller receives the corresponding control instruction, and then controls the uterine cavity distention pump 13 to deliver a first preset amount of liquid into the uterine cavity 4, wherein the first preset amount corresponds to the suction pump 231; and the controller then controls the suction pump 231 to work, driving the liquid in the uterine cavity 4 to enter the waste liquid collection container 22 through the suction tube 21.

### Embodiment 2:

Referring to FIG. 2 of the description, a uterine cavity perfusion system provided by the second preferred embodiment of the present invention is described. The uterine cavity perfusion system of the second preferred embodiment is approximately the same as the first preferred embodiment, except that specific pump forms of the driving member 23 of the suction assembly 2 is different.

Referring to FIG. 2 of the description, the driving member 23 includes a vacuum suction pump 232; the waste liquid collection container 22 includes a second waste liquid collection container 222; the second waste liquid collection container 222 is disposed between the vacuum suction pump 232 and the uterine cavity 4; and the vacuum suction pump 232 is used for sucking a gas in the second waste liquid collection container 222 to generate a negative pressure in the second waste liquid collection container 222 so as to drive the liquid in the uterine cavity 4 to enter.

Referring to FIG. 2 of the description, the suction assembly 2 further includes a filtering member 25 connected to the vacuum suction pump 232.

Preferably, in the present preferred embodiment, the second waste liquid collection container 222 is preferably a waste liquid collection bottle. The vacuum suction pump 232 is controlled to work while the liquid in the uterine cavity 4 needs to be discharged. A gas in the second waste liquid collection container 222 is sucked, thereby forming a negative pressure in the second waste liquid collection container 222 so as to guide the liquid in the uterine cavity 4 to enter the second waste liquid collection container 222 under the action of the gas pressure.

In the working process, a corresponding control instruction is sent to the controller when the liquid in the uterine cavity 4 needs to be discharged; the controller receives the corresponding control construction, and then controls the uterine cavity distention pump 13 to pre-start to inject a second preset amount of liquid into the uterine cavity 4, wherein the second preset amount corresponds to a response speed of the vacuum suction pump 232; and the controller then controls the vacuum suction pump 232 to work to suck a gas in the second waste liquid collection container 222, thereby forming a negative pressure in the second waste liquid collection container 222 so as to drive the liquid in the uterine cavity 4 to enter under the action of the gas pressure.

### Embodiment 3:

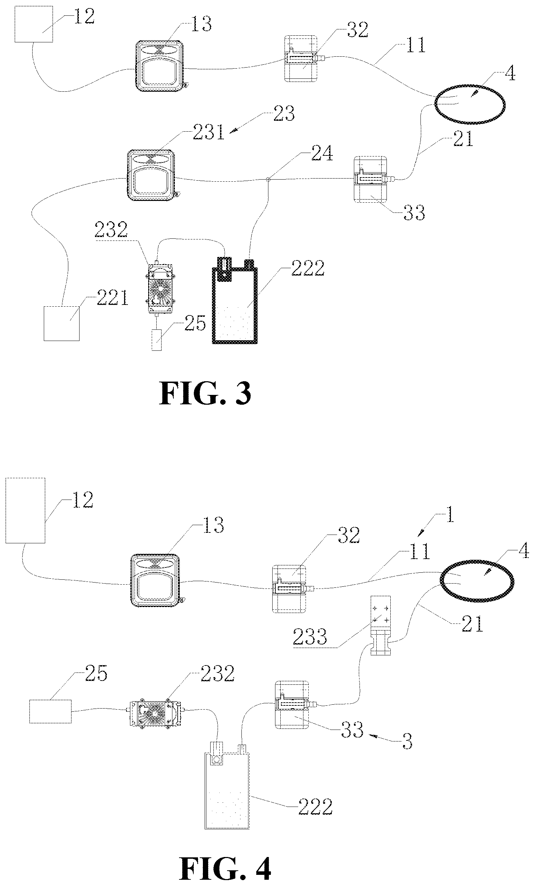

Referring to FIG. 3 of the description, a third preferred embodiment of the uterine cavity perfusion system provided by the present invention is described. The uterine cavity perfusion system provided by the third preferred embodiment is different from the above-described preferred embodiments in that two gas discharge modes for the suction assembly 2 in the first preferred embodiment and the suction assembly 2 in the second preferred embodiment described above are combined to form two liquid discharge paths.

Referring to FIG. 3 of the description, the suction assembly 2 further includes a three-way valve 24, wherein the suction pump 231 is communicated with the three-way valve 24, and the three-way valve 24 is also connected to the second waste liquid collection container 222.

In the present preferred embodiment, the three-way valve 24 is in communication connection to the controller. The controller can control an on-state of the three-way valve 24, such that the liquid in the uterine cavity 4 enters the first waste liquid collection container 221 or the second waste liquid collection container 222.

Exemplarily, in a normal state, the suction pump 231 is connected to the uterine cavity 4, and the second waste liquid collection container 222 is disconnected from the uterine cavity 4, so that the liquid in the uterine cavity 4 enters the first waste liquid collection container 221. When the suction tube 21 is obstructed, the three-way valve 24 is controlled so that the uterine cavity 4 is communicated with the second waste liquid collection container 222, and the liquid in the uterine cavity 4 is driven to flow by a negative pressure in the second waste liquid collection container 222, so that the suction tube 21 releases the obstructed state.

### Embodiment 4:

Referring to FIG. 4 of the description, a fourth preferred embodiment of the uterine cavity perfusion system provided by the present invention is described. The uterine cavity perfusion system of the fourth preferred embodiment is different from the second preferred embodiment in that the driving member 23 of the suction assembly 2 further includes a pinch valve 233.

Referring to FIG. 4 of the description, the pinch valve 233 is connected between the second waste liquid collection container 222 and the uterine cavity 4 and used for controlling the connection and disconnection of the suction tube 21 between the second waste liquid collection container 222 and the uterine cavity 4.

Referring to FIG. 4 of the description, the pinch valve 233 has an opened state and a closed state. When the pinch valve 233 is in the opened state, the liquid in the uterine cavity 4 can enter the second waste liquid collection container 222 through the suction tube 21; and when the pinch valve 233 is in the closed state, the uterine cavity 4 is disconnected from the second waste liquid collection container 222, such that the liquid in the uterine cavity 4 cannot enter the second waste liquid collection container 222.

The pinch valve 233 is also connected to the controller, and the controller can control the pinch valve 233 to switch between the opened state and the closed state. When it is necessary to control the liquid in the uterine cavity 4 to enter the second waste liquid collection container 222, the pinch valve 233 is switched from the closed state to the opened state, and the liquid in the uterine cavity 4 is guided to the second waste liquid collection container 222 by virtue of the negative pressure in the second waste liquid collection container 222.

It should also be pointed out that the vacuum suction pump 232 can form a negative pressure environment more quickly in the second waste liquid collector 222 when the pinch valve 233 is in the closed state.

### Embodiment 5:

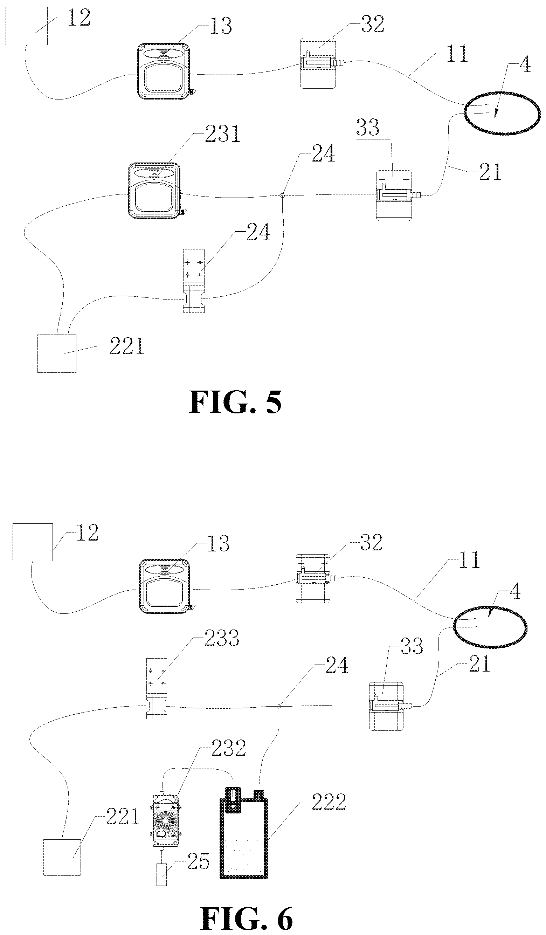

Referring to FIG. 5 of the description, a fifth preferred embodiment of the uterine cavity perfusion system provided by the present invention is described. The uterine cavity perfusion system of the fifth preferred embodiment is different from the third preferred embodiment in that the vacuum suction pump 232 and the second waste liquid collection container 222 are replaced by the pinch valve 233.

Referring to FIG. 5 of the description, one end of the pinch valve 233 is connected to the first waste liquid collection container 221, and the other end of the pinch valve 233 is connected to the three-way valve 24; and the three-way valve 233 is also connected to the suction pump 231.

It should be pointed out that in the present preferred embodiment, when the liquid in the uterine cavity 4 needs to enter the first waste liquid collection container 221, the three-way valve 24 is controlled such that the pinch valve 233 is communicated with the uterine cavity 4, and then the pinch valve 233 is controlled to switch to the opened state, so that the liquid in the uterine cavity 4 enters the first waste liquid collection container 221 by its own gravity. When the three-way valve 24 is controlled such that the suction pump 231 is communicated with the uterine cavity 4, the liquid in the uterine cavity 4 enters the first waste liquid collection container 221 under the action of the suction pump 231.

### Embodiment 6:

Referring to FIG. 6 of the description, a sixth preferred embodiment of the uterine cavity perfusion system provided by the present invention is described. The uterine cavity perfusion system of the sixth preferred embodiment is different from the third preferred embodiment in that the suction pump 231 is replaced by the pinch valve 233.

Specifically, one end of the pinch valve 233 is connected to the first waste liquid collection container 221, and the other end of the pinch valve 233 is connected to the three-way valve 24; and the three-way valve 24 is also connected to the second waste liquid collection container 222.

Exemplarily, the three-way valve 24 is controlled such that the pinch valve 233 is communicated with the uterine cavity 4. When the pinch valve 233 is in an opened state, the liquid in the uterine cavity 4 can enter the first waste liquid collection container 221 through the pinch valve 233. The three-way valve 24 is controlled such that the second waste liquid collection container 222 is communicated with the uterine cavity 4, so that the liquid in the uterine cavity 4 can enter the second waste liquid collection container 222 under the negative pressure in the second waste liquid collection container 222.

### Embodiment 7:

According to a further aspect of the present invention, the present invention further provides a uterine cavity perfusion method, comprising:
injecting a preset amount of liquid into the uterine cavity 4 in advance through a uterine cavity distention assembly 1 while a liquid in the uterine cavity 4 needs to be discharged, and then discharging the liquid in the uterine cavity 4 through a suction assembly 2, wherein the uterine cavity distention assembly 1 and the suction assembly 2 work in cooperation with each other.

That is, in the uterine cavity perfusion method provided by the present preferred embodiment, a preset amount of liquid is injected into the uterine cavity 4 in advance by the uterine cavity distention assembly 1 before the liquid in the uterine cavity 4 is discharged through the suction assembly 2, thereby preventing the collapse of the uterine cavity 4 caused by a sudden decrease in liquid pressure in the uterine cavity 4 while the liquid in the uterine cavity 4 is suddenly discharged.

Further, the uterine cavity perfusion method, the use of the uterine cavity perfusion system according to the Embodiment 1 comprises the following steps:
pressing a liquid discharge button to send a corresponding control instruction to the controller;
controlling, by the controller, the uterine cavity distention pump 13 of the uterine cavity distention assembly 1 to deliver the first preset amount of liquid into the uterine cavity in response to receiving the corresponding control instruction; and
controlling, by the controller, the suction pump 231 of the suction assembly 2 to work after a first preset time, so as to drive the liquid in the uterine cavity to enter the first waste liquid collection container 221 through the suction tube 21.

Further, the uterine cavity perfusion method, the use of the uterine cavity perfusion system according to the Embodiment 2 comprises the following steps:
pressing a liquid discharge button to send a corresponding control instruction to the controller;
controlling, by the controller, the uterine cavity distention pump 13 of the uterine cavity distention assembly 1 to deliver the second preset amount of liquid into the uterine cavity in response to receiving the corresponding control instruction; and
controlling, by the controller, the vacuum suction pump 232 of the suction assembly 2 to work after a second preset time to suck the gas in the second waste liquid collection container 222, thereby forming a negative pressure in the second waste liquid collection container 222 so as to drive the liquid in the uterine cavity to enter under the action of the gas pressure.

It should be noted that the above embodiments may be freely combined as needed. The above description is only preferred embodiments of the present invention, and it should be pointed out that those of ordinary skill in the art may also make several improvements and modifications without departing from the principles of the present invention, which should be considered as the protection scope of the present invention.

## Claims

1. A uterine cavity perfusion system, comprising:
a uterine cavity distention assembly, comprising a uterine cavity distention tube, a saline storage container and a uterine cavity distention pump, wherein the uterine cavity distention tube is connected to the saline storage container and a uterine cavity, and the uterine cavity distention pump is installed on the uterine cavity distention tube and used for driving liquid in the saline storage container to enter the uterine cavity;
a suction assembly, comprising a suction tube, a waste liquid collection container and a driving member, wherein the suction tube is connected to the waste liquid collection container, and the driving member is installed on the suction tube and used for driving liquid in the uterine cavity to enter the waste liquid collection container; and
a control assembly, comprising a controller, wherein the controller is connected to the uterine cavity distention pump and the driving member respectively, and the controller is used for coordinating the work of the uterine cavity distention pump and the driving member so that a pressure of the liquid in the uterine cavity is stable.

2. The uterine cavity perfusion system according to claim 1, wherein the controller, prior to controlling the driving member to drive the liquid in the uterine cavity to leave through the suction tube, controls the uterine cavity distention pump to pre-deliver a predetermined amount of liquid into the uterine cavity, so as to prevent the collapse of the uterine cavity caused by fast outflow of the liquid in the uterine cavity.

3. The uterine cavity perfusion system according to claim 2, wherein the control assembly further comprises a uterine cavity distention pump pressure sensor and a driving member pressure sensor, wherein the uterine cavity distention pump pressure sensor is installed on the uterine cavity distention tube and used for detecting a liquid pressure in the uterine cavity distention tube; and the driving member pressure sensor is installed on the suction tube and used for detecting a liquid pressure in the suction tube.

4. The uterine cavity perfusion system according to claim 3, wherein the driving member comprises a suction pump; the waste liquid collection container comprises a first waste liquid collection container; and the suction pump is disposed between the first waste liquid collection container and the uterine cavity and used for driving the liquid in the suction tube to flow from the uterine cavity to the first waste liquid collection container.

5. The uterine cavity perfusion system according to claim 3, wherein the driving member comprises a vacuum suction pump; the waste liquid collection container comprises a second waste liquid collection container; the second waste liquid collection container is disposed between the vacuum suction pump and the uterine cavity; and the vacuum suction pump is used for sucking a gas in the second waste liquid collection container to generate a negative pressure in the second waste liquid collection container, thereby driving the liquid in the uterine cavity to enter.

6. The uterine cavity perfusion system according to claim 4, wherein the driving member comprises a vacuum suction pump; the waste liquid collection container comprises a second waste liquid collection container; the vacuum suction pump is communicated with the second waste liquid collection container and used for sucking a liquid in the second waste liquid collection container; the suction assembly further comprises a three-way valve; the suction pump is communicated with the three-way valve; and the three-way valve is also connected to the second waste liquid collection container.

7. The uterine cavity perfusion system according to claim 5, wherein the driving member further comprises a pinch valve; and the pinch valve is connected between the second waste liquid collection container and the uterine cavity and used for controlling the connection and disconnection of the suction tube between the second waste liquid collection container and the uterine cavity.

8. The uterine cavity perfusion system according to claim 4, wherein the driving member further comprises a pinch valve; the suction assembly further comprises a three-way valve; one end of the pinch valve is connected to the first waste liquid collection container, and the other end of the pinch valve is connected to the three-way valve; and the three-way valve is also connected to the suction pump.

9. The uterine cavity perfusion system according to claim 5, wherein the driving member further comprises a pinch valve; the waste liquid collection container further comprises a third waste liquid collection container; the suction assembly further comprises a three-way valve; one end of the pinch valve is connected to the third waste liquid collection container, and the other end of the pinch valve is connected to the three-way valve; and the three-way valve is also connected to the second waste liquid collection container.

10. A uterine cavity perfusion method, comprising:
injecting a preset amount of liquid into a uterine cavity in advance through a uterine cavity distention assembly while a liquid in the uterine cavity needs to be discharged, and then discharging the liquid in the uterine cavity through a suction assembly, wherein the uterine cavity distention assembly and the suction assembly work in cooperation with each other.

11. The uterine cavity perfusion method according to claim 10, wherein a corresponding control instruction is sent to a controller while the waste liquid in the uterine cavity needs to be discharged; the controller receives the corresponding control instruction, and then controls a uterine cavity distention pump of the uterine cavity distention assembly to deliver a first preset amount of liquid into the uterine cavity, wherein the first preset amount corresponds to a suction pump of the suction assembly; and the controller then controls the suction pump to work, driving the liquid in the uterine cavity to enter the waste liquid collection container through the suction tube.

12. The uterine cavity perfusion method according to claim 10, wherein the use of the uterine cavity perfusion system according to claim 4 comprises the following steps:
pressing a liquid discharge button to send a corresponding control instruction to the controller;
controlling, by the controller, the uterine cavity distention pump of the uterine cavity distention assembly to deliver the first preset amount of liquid into the uterine cavity in response to receiving the corresponding control instruction; and
controlling, by the controller, the suction pump of the suction assembly to work after a first preset time, so as to drive the liquid in the uterine cavity to enter the first waste liquid collection container through the suction tube.

13. The uterine cavity perfusion method according to claim 10, wherein a corresponding control instruction is sent to the controller when the liquid in the uterine cavity needs to be discharged; the controller receives the corresponding control construction, and then controls the uterine cavity distention pump of the uterine cavity distention assembly to pre-start to inject a second preset amount of liquid into the uterine cavity, wherein the second preset amount corresponds to a response speed of a vacuum suction pump; and the controller then controls the vacuum suction pump to work to suck a gas in a second waste liquid collection container, thereby forming a negative pressure in the second waste liquid collection container so as to drive the liquid in the uterine cavity to enter under the action of the gas pressure.

14. The uterine cavity perfusion method according to claim 10, wherein the use of the uterine cavity perfusion system according to claim 5 comprises the following steps:
pressing a liquid discharge button to send a corresponding control instruction to the controller;
controlling, by the controller, the uterine cavity distention pump of the uterine cavity distention assembly to deliver the second preset amount of liquid into the uterine cavity in response to receiving the corresponding control instruction; and
controlling, by the controller, the vacuum suction pump of the suction assembly to work after a second preset time to suck the gas in the second waste liquid collection container, thereby forming a negative pressure in the second waste liquid collection container so as to drive the liquid in the uterine cavity to enter under the action of the gas pressure.
